# EUROPEAN PATENT SPECIFICATION

(11) **EP 3 454 338 B1**
(45) Date of publication and mention of the grant of the patent: **01.04.2020**
(21) Application number: 17890833.1
(22) Date of filing: 25.07.2017
(51) Int. Cl.: G16H 20/60, A61B 5/00, A61B 5/145

(54) **MEAL ADVICE PROVIDING SYSTEM AND ANALYSIS DEVICE**
SYSTEM ZUR BEREITSTELLUNG VON SPEISENEMPFEHLUNGEN UND ANALYSEVORRICHTUNG
PROCÉDÉ DE FOURNITURE DE CONSEILS EN MATIÈRE DE REPAS ET DISPOSITIF D'ANALYSE

(43) Date of publication of application: 13.03.2019
(73) Proprietor: E3 Co., Ltd., Shinjuku-Ku Tokyo 1630532 (JP)
(72) Inventor: YAO Bingwei, Tokyo 163-0532 (JP)
(74) Representative: Vossius & Partner Patentanwälte Rechtsanwälte mbB
(86) International application number: PCT/JP2017/026753
(87) International publication number: WO 2019/021358

(56) References cited:
- EP-A1- 2 408 349
- WO-A1-2012/019746
- WO-A1-2012/090361
- WO-A1-2014/162549
- WO-A1-2014/162549
- WO-A1-2016/194308
- JP-A- 2017 054 163
- US-A1- 2002 047 867
- US-A1- 2017 128 007

## Description

### Technical Field

The present invention relates to a meal advice provision system and an analysis apparatus. In particular, the present invention is suitable for use in a system that measures and analyzes biological information of a user and provides meal advice in accordance with analysis results thereof.

### Background Art

In the background art, focusing on the relationship between diet and health, systems have been proposed that accumulate captured images of meals and biological information of a subject and analyze the correlation between the two in order to give meal advice (see, for example, PTLs 1 to 3) .

With the meal advice system described in PTL 1, a situation in which a subject is eating is imaged and biological data (body weight, body fat, blood pressure, activity level, sleep, body temperature, blood sugar level, and so on) of the subject is measured. Then, on the basis of the measured biological data and meal record information, generated from the captured images and including a plurality of items related to the meal, relevance data is created by analyzing the relevance between the various items of the meal record information and fluctuations in the biological data. Furthermore, meal-related advice is generated on the basis of the relevance data and the generated advice is provided to the subject.

With the health management instruction system described in PTL 2, a server device accumulates, as historical data, personal data including health information and meal information, determines abnormalities in health information that has been input on the basis of the personal data and threshold data, and gives instruction for meal content on the basis of the personal data. The server device does not only perform biological abnormality determination on the basis of daily measurement data, but also advises on the meal content and gives effective treatment means for conditions such as lifestyle diseases.

With the personal health management system described in PTL 3, personal health management information is extracted on the basis of chronological health information including one or more health data items representing biological information (body fat percentage or the like) obtained by taking measurements of the body of a user of the system and one or more lifestyle data items including a calorie intake from the meal of the user, and this information is presented to the user. Meal content is imaged by a mobile terminal provided with imaging means and the calorie intake from the meal is automatically calculated on the basis of that image data.

PTL 4 describes a portable blood glucose level measurement apparatus that enables ex-post-facto confirmation of a relationship between food and/or drink ingested by a person from which measurements are to be taken and a blood sugar level. Specifically, the blood glucose level measurement apparatus of PTL 4 includes a measurement unit that generates and outputs a signal indicating a blood glucose level, a camera unit that generates and outputs an image signal of an imaged subject (food and/or drink imaged when eating and/or drinking by the person from which measurements are to be taken), and a display that displays the blood glucose level and an image on the basis of the signal indicating the glucose level and the image signal.

PTL 5 describes a blood glucose management system, which is provided with a smartphone that can save a user's blood glucose level information. The smartphone has a blood glucose level information acquisition section for acquiring the blood glucose level information, and an announcement section for, on the basis of the acquisition of the blood glucose level information, performing an announcement that prompts acquisition of meal image information.

PTL 6 describes analyzing an image associated with a meal and determining an estimate of a nutritional parameter of the food.

PTL 7 describes a method of processing analyte data using an in vivo analyte monitoring system comprising a sensor control device and a reader device.

### Citation List

### Patent Literature

PTL 1: JP-A-2017-54163
PTL 2: JP-A-2004-201987
PTL 3: JP-A-2006-155010
PTL 4: JP-A-2011-117911
PTL 5: WO 2014/162549 A1
PTL 6: WO 2012/019746 A1
PTL 7: US 2017/128007 A1

### Disclosure of the Invention

However, the systems described in PTLs 3 and 4 merely display and present the relationship between the meal and the biological information to the user, and do not provide the user with meal-related advice. Moreover, while the system described in PTL 2 determines biological abnormalities on the basis of daily measurement data and gives advice for meal content, this system merely determines that there is an abnormality when a measurement value exceeds a threshold, and does not analyze fluctuations in the measurement values.

In contrast, with the system described in PTL 1, the relationship between the various items (information related to meal content (dish name), meal volume, meal time, total eating time, meal speed, meal order, and whether or not alcohol was consumed (and type of alcohol)) of the meal record information and fluctuations in the biological data (body weight, body fat, blood pressure, activity level, sleep, body temperature, blood sugar level, and so on) are analyzed and meal-related advice is generated on the basis of these analysis results.

However, with the system described in PTL 1, the appearance of the meal is imaged by a camera fixed to the ceiling and the captured video is analyzed in order to acquire the meal record information. As such, at locations where a camera is not installed on the ceiling, it is not possible to acquire the meal record information and, consequently, meal-related advice cannot be generated and provided to the user.

With the system described in PTL 1, the actual effects of the content of and manner of eating dinner and the like from the previous day on the biological data (body weight and blood sugar level) from the morning of the present day is analyzed by acquiring the meal record information for a meal (dinner) of the previous day, and calculating a difference between the biological data from the morning of the previous day and biological data from the morning of the present day. Then, meal advice is provided to the subject on the basis of analysis results thereof.

However, there is a possibility that the user participated in various activities and meals other than the dinner of the previous day between the morning of the previous day and the morning of the present day. As such, there is no clear causal relationship between the dinner of the previous day and the fluctuation in the biological data from the previous day to the present day. Therefore, there is a possibility that the analysis results are inaccurate and that appropriate meal advice cannot be provided to the user.

In light of these problems, an object of the present invention is to provide a user with appropriate meal advice based on analysis results of fluctuations in biological information when eating at any location, not only at special locations where a camera is installed on the ceiling.

To solve the problems described above, a meal advice provision system of the invention includes a biological information measurement apparatus that measures biological information of a user, and an analysis apparatus that receives the biological information measured by the biological information measurement apparatus and analyses the biological information. The analysis apparatus analyzes a change between pre-meal and post-meal biological information on the basis of pre-meal and post-meal biological information measured by the biological information measurement apparatus and times at which the pre-meal and post-meal biological information were obtained, and provides meal-related advice on the basis of analysis results thereof. The present invention is defined in the claims.

With the invention configured as described above, the meal-related advice is provided on the basis of the change between the pre-meal and post-meal biological information. As such, the meal-related advice can be provided even when there is no captured image of the meal. Additionally, an analysis is conducted using the pre-meal and post-meal biological information and the meal-related advice is provided on the basis of the analysis results thereof. As such, the causal relationship between the meal and the fluctuation between the pre-meal and post-meal biological information is clear. As a result, with this invention, a user can be provided with appropriate meal advice based on the analysis results of the fluctuation in the biological information when eating at any location, not only at special locations where a camera is installed on the ceiling.

### Brief Description of the Drawings

Fig. 1 is a drawing illustrating an example of a configuration of a meal advice provision system according to a first embodiment;
Fig. 2 is a block diagram illustrating an example of functional components of an analysis apparatus according to the first embodiment;
Fig. 3 is a flowchart illustrating an example of operations of the meal advice provision system according to the first embodiment;
Fig. 4 is a block diagram illustrating an example of functional components of an analysis apparatus according to a second embodiment;
Fig. 5 is a flowchart illustrating an example of operations of a meal advice provision system according to a second embodiment;
Fig. 6 is a drawing illustrating an example of a configuration of a meal advice provision system according to a third embodiment;
Fig. 7 is a block diagram illustrating an example of functional components of an analysis apparatus according to the third embodiment;
Fig. 8 is a flowchart illustrating an example of operations of the meal advice provision system according to the third embodiment;
Fig. 9 is a flowchart illustrating an example of the operations of the meal advice provision system according to the third embodiment; and
Fig. 10 is a flowchart illustrating an example of the operations of the meal advice provision system according to the third embodiment.

### Best Mode for Carrying Out the Invention

### First Embodiment

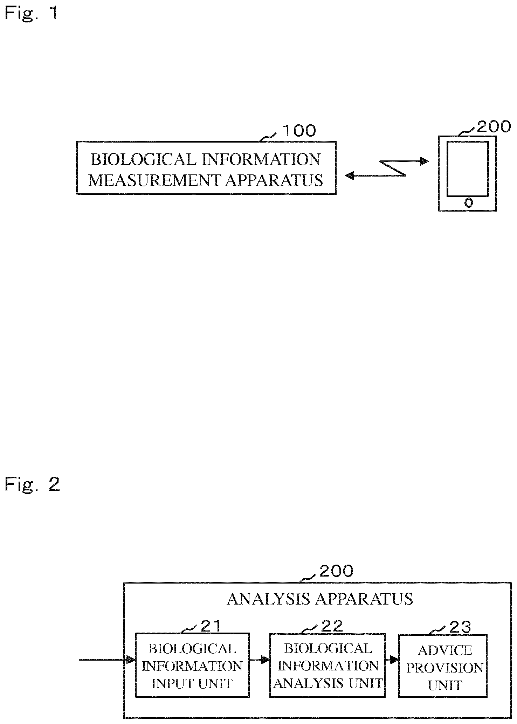

Next, a first embodiment according to the invention will be described while referencing the drawings. Fig. 1 is a drawing illustrating an example of a configuration of a meal advice provision system according to the first embodiment. As illustrated in Fig. 1, the meal advice provision system according to the first embodiment includes a biological information measurement apparatus 100 that measures biological information of a user, and an analysis apparatus 200 that receives the biological information measured by the biological information measurement apparatus 100 and analyses the biological information.

The biological information measurement apparatus 100 is connected to the analysis apparatus 200 by wireless communication means such as Bluetooth (registered trademark) or a wireless LAN, or wired communication means such as a universal serial bus (USB).

In the first embodiment, the biological information measurement apparatus 100 is a portable mobile device and measures a blood sugar level of the user as the biological information. In one example, the biological information measurement apparatus 100 has a plurality of sensors and detects, using the sensors, data needed to calculate the blood sugar level from blood extracted by pricking a part of the body of the user, such as a finger, with a needle. Then, the biological information measurement apparatus 100 calculates the blood sugar level of the user using various types of detected data.

Specifically, the biological information measurement apparatus 100 calculates a predetermined carbohydrate index indicating the blood sugar level of the user using the correlation existing between the heat release amount emitted when the glucose in the blood is oxidized (metabolic heat) and the oxygen supply amount (blood flow velocity, blood oxygen saturation level) by an calculation algorithm based on the metabolic heat confirmation (MHC) method, which is a noninvasive blood sugar level measurement method.

The analysis apparatus 200 is a portable mobile device and, in one example, is configured from a smartphone. An application program (hereinafter referred to as the "application") is installed on the analysis apparatus 200. The application program receives and analyzes the biological information (the carbohydrate index) measured by the biological information measurement apparatus 100 and gives meal-related advice on the basis of the analysis results (hereinafter referred to as "meal advice").

Note that the analysis apparatus 200 may be configured from a server on the Internet that is connected to the smartphone. In one example, the biological information measured by the biological information measurement apparatus 100 may be input into the smartphone and the biological information may be sent from the smartphone to the server (the analysis apparatus) across the Internet. Then, in the server, the biological information may be analyzed and the meal advice may be generated on the basis of the analysis results, and the generated meal advice may be provided to the smartphone.

Fig. 2 is a block diagram illustrating an example of the functional components of the analysis apparatus 200 according to the first embodiment. Note that Fig. 2 illustrates an example of functional components of a case in which the analysis apparatus 200 is a smartphone. As illustrated in Fig. 2, the analysis apparatus 200 according to the first embodiment includes, as functional components thereof, a biological information input unit 21, a biological information analysis unit 22, and an advice provision unit 23.

The various functional blocks 21 to 23 are realized by an application installed on the analysis apparatus 200. Specifically, in actuality, the various functional blocks 21 to 23 are configured to include a CPU, RAM, ROM, and the like of the analysis apparatus 200, and are realized by operations of an application stored in a storage medium such as RAM, ROM, a hard disk, or semiconductor memory.

The biological information input unit 21 inputs the biological information (the carbohydrate index) measured before a meal and after the meal by the biological information measurement apparatus 100. Specifically, in this embodiment, the user uses the biological information measurement apparatus 100 to measure the biological information prior to eating the meal and also uses the biological information measurement apparatus 100 to measure the biological information after eating the meal. The biological information input unit 21 inputs this biological information measured before and after the meal from the biological information measurement apparatus 100.

The biological information analysis unit 22 analyzes a change between the pre-meal and post-meal biological information on the basis of the pre-meal and post-meal biological information input by the biological information input unit 21 and times at which the pre-meal and post-meal biological information were obtained. Here, the times at which the pre-meal and post-meal biological information were obtained are times at which the biological information is measured by the biological information measurement apparatus 100, and the biological information input unit 21 inputs measurement time information together with the biological information from the biological information measurement apparatus 100.

In one example, a configuration is possible in which the pre-meal and post-meal biological information measured by the biological information measurement apparatus 100 is stored in internal memory and, thereafter, the user operates a send button provided on the biological information measurement apparatus 100 or the analysis apparatus 200, thereby sending the pre-meal and post-meal biological information from the biological information measurement apparatus 100 to the analysis apparatus 200.

In such a case, the biological information measurement apparatus 100 stores the measurement time information together with the biological information measured before and after the meal in the internal memory and sends the measurement time information together with the biological information in accordance with a subsequent operation of a send button. The biological information input unit 21 of the analysis apparatus 200 inputs the measurement time information together with the biological information. Then, the biological information analysis unit 22 analyzes the change between the pre-meal and post-meal biological information on the basis of the pre-meal and post-meal biological information and the measurement time information input by the biological information input unit 21.

Note that a configuration is possible in which the measured biological information is automatically sent to the analysis apparatus 200 each time the biological information measurement apparatus 100 measures the biological information. In such a case, the measurement time information may be sent together with the biological information or the measurement time information may not be sent. When the measurement time information is not sent, the biological information analysis unit 22 regards the time at which the biological information input unit 21 inputs the biological information as the measurement time, and analyzes the change between the pre-meal and post-meal biological information on the basis of the pre-meal and post-meal biological information and the measurement time information.

The items that the biological information analysis unit 22 analyzes are the magnitude of the gradient of the change between the pre-meal and post-meal biological information (the carbohydrate index), the magnitude of the amount of the change, and the like. For example, when the gradient is steep, it can be analyzed that there is a high possibility that the meal was eaten in a short period of time (fast eating without proper chewing). Additionally, when the amount of the change is great, it can be analyzed that there is a high possibility that a large amount of food and/or drink was ingested (overeating).

The advice provision unit 23 provides the meal advice on the basis of the analysis results from the biological information analysis unit 22. The meal advice may be provided by a method in which, for example, the meal advice is displayed on a display of the analysis apparatus 200, namely the smartphone.

Specifically, when the gradient of the change between the pre-meal and post-meal carbohydrate indexes is steeper than a predetermined angle and the biological information analysis unit 22 analyzes that there is a high possibility of fast eating, the advice provision unit 23 displays meal advice such as, "chew your food well", or the like on the display. At the same time, the pre-meal and post-meal carbohydrate indexes may be graphed so as to visualize and display the magnitude of the gradient, thereby calling the user to attention.

Additionally, when the amount of the change between the pre-meal and post-meal carbohydrate indexes is greater than a predetermined value and the biological information analysis unit 22 analyzes that there is a high possibility of overeating, the advice provision unit 23 displays meal advice such as, "watch your portion size", or the like on the display. At the same time, the pre-meal and post-meal carbohydrate indexes may be graphed so as to visualize and display the magnitude of the amount of the change, thereby calling the user to attention.

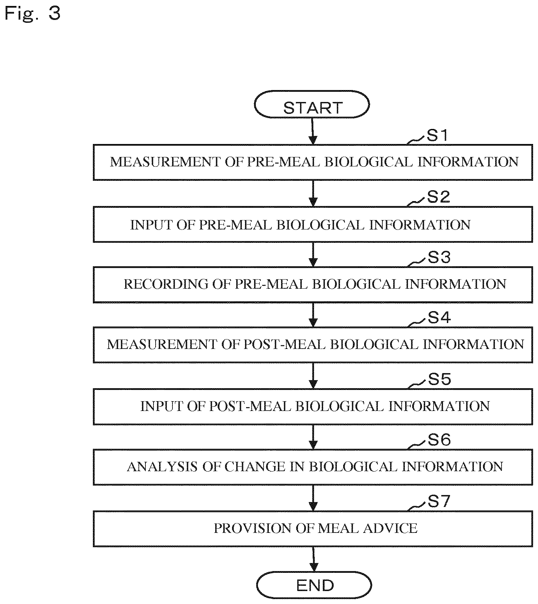

Fig. 3 is a flowchart illustrating an example of the operations of the meal advice provision system according to the first embodiment, having the configuration described above. Note that the flowchart illustrated in Fig. 3 starts when the biological information measurement apparatus 100 is powered ON and the application installed on the analysis apparatus 200 (smartphone) is launched.

First, before the meal, the biological information measurement apparatus 100 measures the carbohydrate index as the biological information of the user (step S1). Specifically, the biological information measurement apparatus 100 detects, via the sensors, data needed to calculate the carbohydrate index, and calculates the carbohydrate index of the user using the various types of detected data.

Note that, before taking this measurement, the user selects "pre-meal" on a meal state selection screen provided by the application of the analysis apparatus 200. As a result, the application will be able to recognize that the carbohydrate index measured by the biological information measurement apparatus 100 is the pre-meal carbohydrate index.

Next, the biological information input unit 21 of the analysis apparatus 200 inputs the pre-meal carbohydrate index measured by the biological information measurement apparatus 100 (step S2). As described above, the user has selected "pre-meal" on the meal state selection screen before measuring the carbohydrate index. As such, in the biological information input unit 21, the carbohydrate index input from the biological information measurement apparatus 100 is recognized as the pre-meal carbohydrate index.

The biological information analysis unit 22 stores the pre-meal carbohydrate index input by the biological information input unit 21 together with the measurement time information of the carbohydrate index in the internal memory (step S3).

After the user has finished the meal, the biological information measurement apparatus 100 measures the carbohydrate index as the biological information of the user (step S4). Before taking this measurement, the user selects "post-meal" on the meal state selection screen provided by the application of the analysis apparatus 200. As a result, the application will be able to recognize that the carbohydrate index measured by the biological information measurement apparatus 100 is the post-meal carbohydrate index.

Next, the biological information input unit 21 of the analysis apparatus 200 inputs the post-meal carbohydrate index measured by the biological information measurement apparatus 100 (step S5). As described above, the user has selected "post-meal" on the meal state selection screen before measuring the carbohydrate index. As such, in the biological information input unit 21, the carbohydrate index input from the biological information measurement apparatus 100 is recognized as the post-meal carbohydrate index.

Next, the biological information analysis unit 22 analyzes the change between the pre-meal and post-meal biological information on the basis of the pre-meal and post-meal biological information input by the biological information input unit 21 and the measurement time information of the pre-meal and post-meal biological information (step S6). Then, on the basis of the analysis results from the biological information analysis unit 22, the advice provision unit 23 displays the meal advice on the display of the analysis apparatus 200, thereby providing the meal advice to the user (step S7).

As described in detail above, in the first embodiment, the biological information measurement apparatus 100 that measures the biological information of the user and the analysis apparatus 200 that receives the biological information measured by the biological information measurement apparatus and analyses the biological information are provided. The analysis apparatus 200 analyzes the change between the pre-meal and post-meal biological information on the basis of the pre-meal and post-meal biological information measured by the biological information measurement apparatus 100 and the times at which the pre-meal and post-meal biological information were obtained, and provides the meal advice on the basis of the analysis results thereof.

According to the first embodiment having this configuration, the meal advice is provided on the basis of the change between the pre-meal and post-meal biological information. As such, the meal advice can be provided even when there is no captured image of the meal. Additionally, the analysis is conducted using the pre-meal and post-meal biological information and the meal advice is provided on the basis of the analysis results thereof. As such, the causal relationship between the meal and the fluctuation between the pre-meal and post-meal biological information is clear. Thus, according to the first embodiment, the user can be provided with appropriate meal advice based on the analysis results of the fluctuation in the biological information when eating at any location, not only at special locations where a camera is installed on the ceiling.

### Second Embodiment

Next, a second embodiment according to the invention will be described while referencing the drawings. In the first embodiment described above, the user itself launches the application to perform the measurement and analysis of the biological information. In contrast, in the second embodiment, the application prompts the user to execute the measurement and analysis of the biological information.

The overall configuration of the meal advice provision system according to second embodiment is the same as illustrated in Fig. 1. Note that, as in the first embodiment, the analysis apparatus 200 may be configured from a smartphone, or may be configured from a server on the Internet that is connected to the smartphone. The smartphone is provided with a camera (imaging device) as standard equipment.

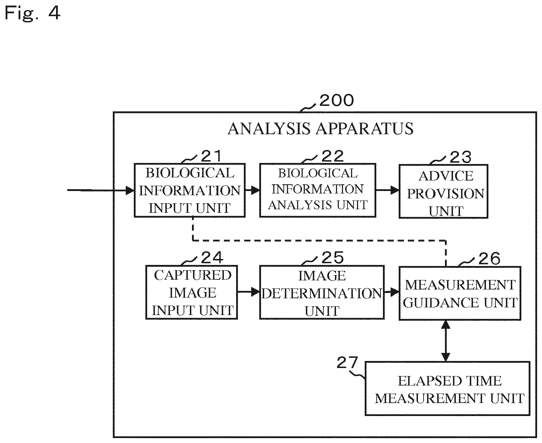

Fig. 4 is a block diagram illustrating an example of the functional components of the analysis apparatus 200 according to the second embodiment. Note that Fig. 4 illustrates an example of the functional components for a case in which the analysis apparatus 200 is a smartphone. In Fig. 4, the components that have the same functions as those in Fig. 2 are marked with the same reference numerals and redundant descriptions thereof are foregone.

As illustrated in Fig. 4, in addition to the configuration illustrated in Fig. 2, the analysis apparatus 200 according to the second embodiment includes, as functional components thereof, a captured image input unit 24, an image determination unit 25, a measurement guidance unit 26, and an elapsed time measurement unit 27. These various functional blocks 24 to 27 are also realized by the application installed on the analysis apparatus 200.

Note that the various processing of the captured image input unit 24, the image determination unit 25, the measurement guidance unit 26, and the elapsed time measurement unit 27 are executed in the background prior to the user operating the start up button (icon) to launch the application. In contrast, the various processing of the biological information input unit 21, the biological information analysis unit 22, and the advice provision unit 23 are executed after the user operates the start up button (icon) to launch the application.

The captured image input unit 24 inputs a captured image captured by the camera of the smartphone. Note that the captured image input here is not limited to an image captured of a meal. When the user operates the camera to capture an image, that captured image is input by the captured image input unit 24. The captured images input by the captured image input unit 24 also include images of meals captured by the user to commemorate something, images of meals captured to upload to social network services (SNS), and the like.

The image determination unit 25 analyzes the captured images input by the captured image input unit 24 to determine whether the captured images that have been input are images of meals. Here, the analysis of the captured images is, for example, image recognition processing. In one example, it is determined whether a captured image that has been input is an image of a meal by comparing a feature quantity extracted from the captured image input by the captured image input unit 24 against a feature quantity of a meal image registered in advance.

Note that the method for determining whether the captured image that has been input is an image of a meal is not limited thereto, and various known methods may be used. For example, a configuration is possible in which it is determined whether the captured image that has been input is an image of a meal by inputting the captured image input by the captured image input unit 24 into a learning model generated by learning a plurality of images of meals.

The measurement guidance unit 26 notifies a message prompting the measurement of the biological information when the image determination unit 25 determines that the captured image is an image of a meal. The message may be notified by a method in which, for example, the message is displayed on the display of the analysis apparatus 200, namely the smartphone.

Here, when the image determination unit 25 determines that the captured image is an image of a meal, the measurement guidance unit 26 notifies the message when the determination is made and preferably notifies the message again after a predetermined amount of time. The predetermined amount of time is the time it is expected to take to finish the meal, for example, 1 hour. The elapsed time measurement unit 27 measures this elapsed time of 1 hour.

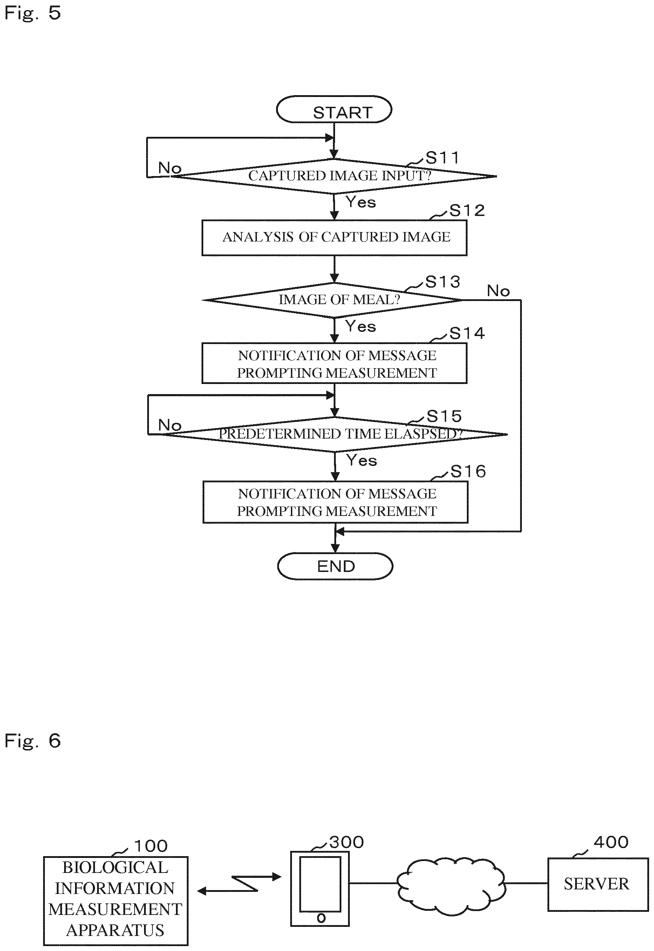

Fig. 5 is a flowchart illustrating an example of the operations executed in the background by the application installed on the analysis apparatus 200 in the second embodiment having the configuration described above. The flowchart illustrated in Fig. 5 is constantly running in the background while the analysis apparatus 200 (the smartphone) is powered ON.

First, the captured image input unit 24 determines whether an image has been captured by the camera of the smartphone, that is, determines whether the captured image has been input (step S11). When the captured image input unit 24 has input the captured image, the image determination unit 25 analyzes the captured image input by the captured image input unit 24 and determines whether the captured image that has been input is an image of a meal (step S13).

Here, when the image determination unit 25 determines that the captured image is not an image of a meal, the processing of the flowchart illustrated in Fig. 5 is ended. Meanwhile, when the image determination unit 25 determines that the captured image is an image of a meal, the measurement guidance unit 26 notifies the user by displaying the message prompting the measurement of the biological information on the display of the analysis apparatus 200 (step S14). Upon receipt of the notification of this message, the user launches the application by operating the start up button of the application, and performs the measurement of the biological information in accordance with the procedures of steps S1 to S3 illustrated in Fig. 3.

After the measurement guidance unit 26 has notified the message in step S14, the elapsed time measurement unit 27 measures the elapsed time and determines whether the predetermined amount of time (e.g. 1 hour) has elapsed (step S15). When the predetermined amount of time has elapsed, the measurement guidance unit 26 notifies the user by displaying the message prompting the measurement of the biological information on the display of the analysis apparatus 200 (step S16). Upon receipt of the notification of this message, the user performs the measurement of the biological information in accordance with the procedures of steps S4 to S5 illustrated in Fig. 3.

Then, the biological information analysis unit 22 analyzes the change between the pre-meal and post-meal biological information on the basis of the pre-meal biological information that the user measured using the biological information input unit 100 as a result of being prompted by the first message in step S14, the post-meal biological information that the user measured using the biological information input unit 100 as a result of being prompted by the second message in step S16, and the measurement time information of the pre-meal and post-meal biological information (step S6 of Fig. 3). Then, on the basis of the analysis results from the biological information analysis unit 22, the advice provision unit 23 displays the meal advice on the display of the analysis apparatus 200, thereby providing the meal advice to the user (step S7 of Fig. 3).

Note that in the second embodiment, in cases where the user performs the measurement of the biological information, it is not necessary to select "pre-meal" and "post-meal" on a meal state selection screen. This is because, in the second embodiment, the measurement guidance unit 26 notifies the message prompting the measurement of the biological information two times and the biological information input unit 21 inputs the biological information in accordance therewith. As such, the application itself is capable of ascertaining if the biological information to be input is "pre-meal" or "post-meal" biological information.

As described in detail above, according to the second embodiment, the message prompting the measurement of the biological information is notified when the user captures an image of a meal for some purpose, even if the user is not aware of the measuring of the biological information or the user forgets to measure the biological information. As a result, the user can perform the measurements of the biological information and obtain the meal advice. Additionally, in the second embodiment, the message is notified not only when the user captures an image of a meal (pre-meal), but also after the predetermined amount of time when the meal is expected to be finished. As such, the user will not forget to measure the post-meal biological information and can obtain the meal advice.

### Third Embodiment

Next, a third embodiment according to the invention will be described while referencing the drawings. The third embodiment has a configuration in which, in addition to the application prompting the user to execute the measurement and analysis of the biological information when an image of a meal is captured as in the second embodiment, the image of the meal is also subjected to the analysis.

Fig. 6 is a drawing illustrating an example of a configuration of the meal advice provision system according to the third embodiment. As illustrated in Fig. 6, the meal advice provision system according to the third embodiment includes a biological information measurement apparatus 100 that measures biological information of a user, a mobile terminal 300 that sends the biological information measured by the biological information measurement apparatus 100 and an image of a meal captured by a camera to a server 400, and a server 400 that analyzes the information received from the mobile terminal 300 to provide meal advice. In the third embodiment, the mobile terminal 300 and the server 400 correspond to the analysis apparatus recited in the claims.

The biological information measurement apparatus 100 is connected to the mobile terminal 300 by wireless communication means such as Bluetooth (registered trademark) or a wireless LAN, or wired communication means such as a USB. Additionally, the mobile terminal 300 is connected to the server 400 via the Internet or similar communications network.

In the third embodiment, the biological information measurement apparatus 100 is a portable mobile device and measures a carbohydrate index of the user as the biological information. In one example, the mobile terminal 300 is configured from a smartphone, is provided with functions to communicate with the biological information measurement apparatus 100 and the server 400, and is provided with a camera (imaging device) as standard equipment.

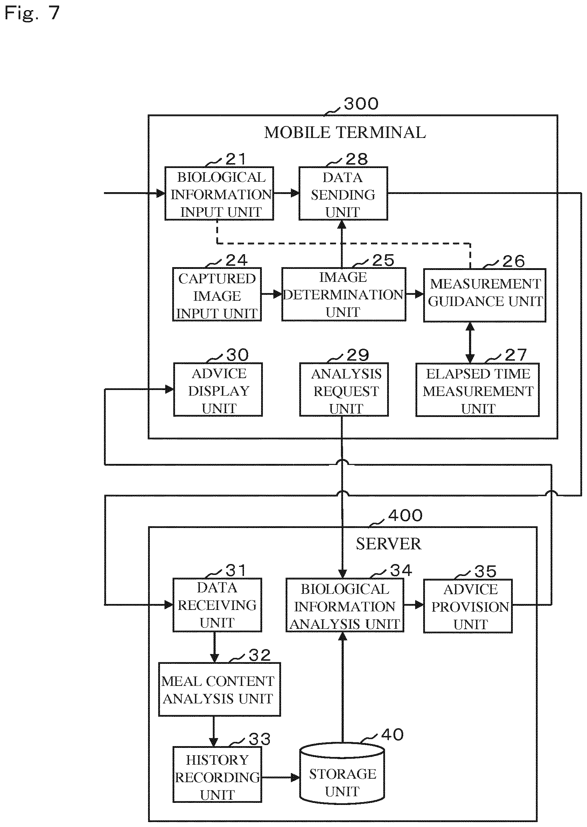

Fig. 7 is a block diagram illustrating an example of the functional components of the analysis apparatus (the mobile terminal 300 and the server 400) according to the third embodiment. In Fig. 7, the components that have the same functions as those in Fig. 4 are marked with the same reference numerals and redundant descriptions thereof are foregone.

As illustrated in Fig. 7, the mobile terminal 300 according to the third embodiment includes, as functional components thereof, the biological information input unit 21, the captured image input unit 24, the image determination unit 25, the measurement guidance unit 26, and the elapsed time measurement unit 27 as illustrated in Fig. 4 and, also, a data sending unit 28, an analysis request unit 29, and an advice display unit 30. Note that the server 400 is provided with functions corresponding to the biological information analysis unit 22 and the advice provision unit 23 illustrated in Fig. 4.

The data sending unit 28 sends the biological information input by the biological information input unit 21 together with the measurement time information thereof to the server 400. Additionally, when the image determination unit 25 determines that the captured image input by the captured image input unit 24 is an image of a meal, the data sending unit 28 sends the image of that meal to the server 400.

In accordance with a user operation, the analysis request unit 29 issues a request, to the server 400, for analyses on the biological information and the image of the meal sent from the mobile terminal 300 to the server 400. As described later, the biological information and the image of the meal sent from the data sending unit 28 to the server 400 are accumulated in the server 400 as historical data. It is possible for the user to send, at a preferred timing, an analysis request to the server 400 by launching the application installed on the mobile terminal 300 and operating an analysis request button.

The advice display unit 30 receives information of the meal advice sent from the server 400 in response to the analysis request being sent from the analysis request unit 29 to the server 400, and displays the meal advice on the display.

Additionally, as illustrated in Fig. 7, the server 400 according to the third embodiment includes, as functional components thereof, a data receiving unit 31, a meal content analysis unit 32, a history recording unit 33, a biological information analysis unit 34, and an advice provision unit 35. Moreover, the server 400 includes a storage unit 40 as a storage medium.

The various functional blocks 31 to 35 can be configured from any of hardware, a digital signal processor (DSP), or software. In one example, when configuring from software, in actuality, the various functional blocks 31 to 35 are configured to include a CPU, RAM, ROM, and the like of a computer, and are realized by the operation of a program stored in a storage medium such as RAM, ROM, a hard disk, or semiconductor memory.

The data receiving unit 31 receives data sent by the data sending unit 28. Specifically, the data receiving unit 31 receives the pre-meal and post-meal biological information input by the biological information input unit 21 of the mobile terminal 300, the time information at which the pre-meal and post-meal biological information were obtained, and the captured image of the meal captured by the camera of the mobile terminal 300.

The meal content analysis unit 32 analyzes meal content from the captured image of the meal received by the data receiving unit 31. Specifically, when the image determination unit 25 of the mobile terminal 300 determines that the captured image is an image of a meal, the meal content analysis unit 32 analyzes the meal content from the captured image of the meal sent from the mobile terminal 300.

The image determination unit 25 of the mobile terminal 300 only determines whether the image captured by the camera is an image of a meal. In contrast, the meal content analysis unit 32 goes as far as to analyze the meal content from the captured image of the meal. This analysis can also be performed by, for example, image recognition processing or machine learning processing.

The history recording unit 33 associates and stores the pre-meal and post-meal biological information received by the data receiving unit 31 (the biological information input by the biological information input unit 21), the measurement time information at which the pre-meal and post-meal biological information were obtained, and the meal content analyzed by the meal content analysis unit 32 as historical data in the storage unit 40. The history recording unit 33 sequentially stores the historical data in the storage unit 40 each time the data receiving unit 31 receives the biological information, the measurement time information, and image information of a meal.

When the analysis request is received from the analysis request unit 29, the biological information analysis unit 34 analyzes a causal relationship between the meal content and the change between the pre-meal and post-meal biological information on the basis of the historical data stored in the storage unit 40 at that time. For example, the biological information analysis unit 34 analyzes the causal relationship between the meal content and the magnitude of the gradient of the change between the pre-meal and post-meal carbohydrate indexes. In one example, the tendency of the size of the gradient that will result from a particular meal content is analyzed. Additionally, meal content with a strong tendency for producing a gradient steeper than a predetermined angle may be analyzed.

Additionally, the biological information analysis unit 34 analyzes the causal relationship between the meal content and the degree of the amount of change between the pre-meal and post-meal carbohydrate indexes. In one example, the degree of the amount of change of the carbohydrate index that will result from a particular meal content is analyzed. Additionally, meal content with a strong tendency for increasing the amount of change of the carbohydrate index greater than a predetermined value may be analyzed.

The advice provision unit 35 provides the meal advice on the basis of the analysis results from the biological information analysis unit 34. The meal advice may be provided by a method in which the meal advice is displayed on a display of the mobile terminal 300. Specifically, the advice display unit 30 displays the meal advice on the display of the mobile terminal 300 by information of the meal advice being generated in the advice provision unit 35 and this information being sent to the mobile terminal 300.

In one example, the advice provision unit 35 displays meal advice such as, "When eating OO (specific meal content), carbohydrates may increase rapidly. Be sure to chew your food well" on the display of the mobile terminal 300. At the same time, the gradient of the change between the pre-meal and post-meal carbohydrates (historical average or the like) of that specific meal content may be graphed so as to visualize and display the magnitude of the gradient, thereby calling the user to attention.

Additionally, the advice provision unit 35 displays meal advice such as, "When eating OO (specific meal content), carbohydrates may increase greatly. Be sure not to overeat" on the display. At the same time, the amount of change between the pre-meal and post-meal carbohydrates (historical average or the like) may be graphed so as to visualize and display the magnitude of the amount of change, thereby calling the user to attention.

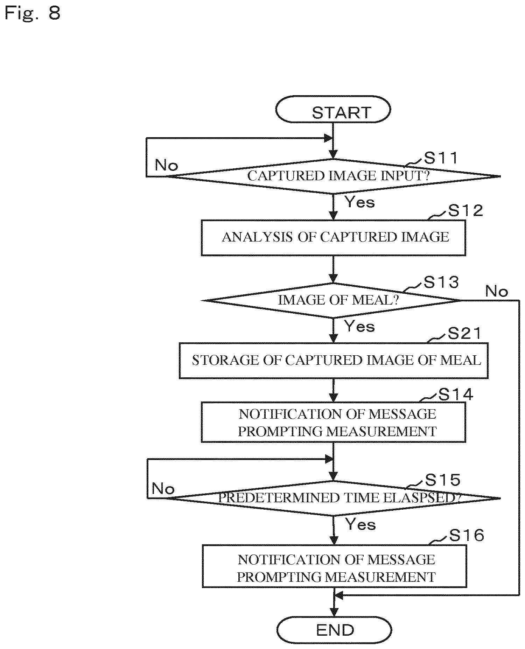

Fig. 8 is a flowchart illustrating an example of the operations of the mobile terminal 300 according to the third embodiment, having the configuration described above. Note that the flowchart illustrated in Fig. 8 depicts an example of the operations to analyze the captured image and notify the message prompting the measurement of the biological information. The operations are executed in the background by the application installed on the mobile terminal 300, and continuously run in the background while the mobile terminal 300 is powered ON.

Additionally, in Fig. 8, steps marked with the same step numbers as in Fig. 5 demonstrate the same processing, and redundant descriptions thereof are foregone. In Fig. 8, when the image determination unit 25 determines that the captured image input by the captured image input unit 24 is an image of a meal (step S13: Yes), the image determination unit 25 stores the image of that meal in the internal memory of the mobile terminal 300. Thereafter, the processing of step S14 is executed and the same processing as that in Fig. 5 is executed.

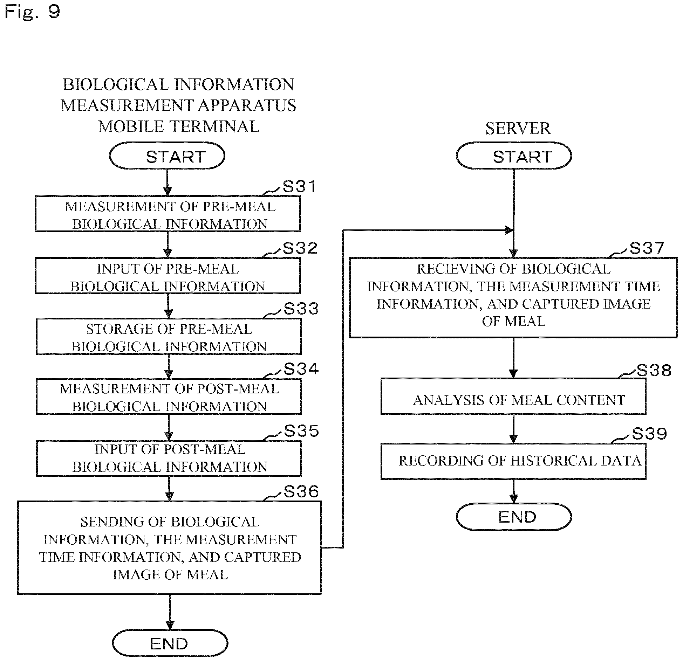

Fig. 9 is a flowchart illustrating an example of the operations of the biological information measurement apparatus 100, the mobile terminal 300, and the server 400 according to the third embodiment. Note that the flowchart illustrated in Fig. 9 depicts an example of the operations to measure the biological information and store the historical data in the server 400. Note that, the flowchart illustrated in Fig. 9 starts when the biological information measurement apparatus 100 is powered ON and the application installed on the mobile terminal 300 is launched.

First, before the meal, the biological information measurement apparatus 100 measures the carbohydrate index as the biological information of the user (step S31). This measurement is performed as a result of being prompted by the first message notified in step S14 of Fig. 8. Accordingly, before taking this measurement, it is not necessary for the user to perform the operation of selecting "pre-meal" on the meal state selection screen.

Next, the biological information input unit 21 of the mobile terminal 300 inputs the pre-meal carbohydrate index measured by the biological information measurement apparatus 100 (step S32). The biological information analysis unit 22 stores the pre-meal carbohydrate index input by the biological information input unit 21 together with the measurement time information of the carbohydrate index in the internal memory (step S33).

After the user has finished the meal, the biological information measurement apparatus 100 measures the carbohydrate index as the biological information of the user (step S34). This measurement is performed as a result of being prompted by the second message notified in step S16 of Fig. 8. Accordingly, before taking this measurement, it is not necessary for the user to perform the operation of selecting "post-meal" on the meal state selection screen.

Next, the biological information input unit 21 of the mobile terminal 300 inputs the post-meal carbohydrate index measured by the biological information measurement apparatus 100 (step S35). Next, the data sending unit 28 sends the pre-meal and post-meal biological information input by the biological information input unit 21, the measurement time information of the pre-meal and post-meal biological information, and the captured image of the meal that was stored in the internal memory in step S21 of Fig. 8 to the server 400 (step S36).

In the server 400, the data receiving unit 31 receives the data (the pre-meal and post-meal biological information, the measurement time information of the biological information, and the captured image of the meal) sent by the data sending unit 28 (step S37). Then, the meal content analysis unit 32 analyzes the meal content from the captured image of the meal received by the data receiving unit 31 (step S38).

Next, the history recording unit 33 associates and stores the pre-meal and post-meal biological information received by the data receiving unit 31, the measurement time information at which the pre-meal and post-meal biological information were obtained, and the meal content analyzed by the meal content analysis unit 32 as historical data in the storage unit 40 (step S39). Thus, the processing of the flowchart illustrated in Fig. 9 is ended.

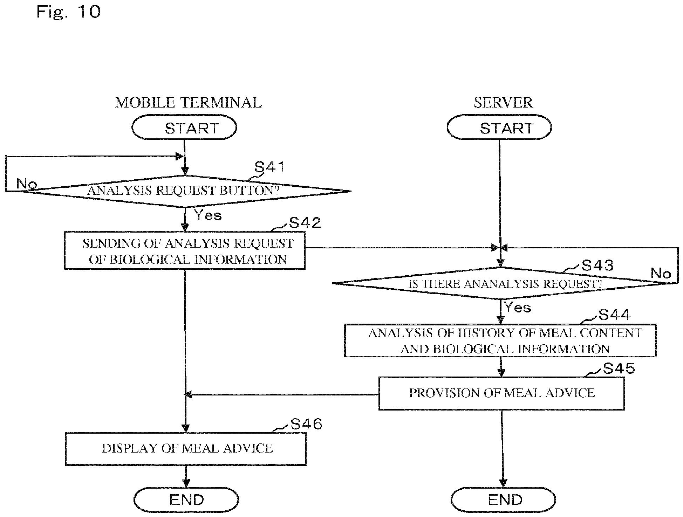

Fig. 10 is a flowchart illustrating an example of the operations of the mobile terminal 300 and the server 400 according to a third embodiment. The flowchart illustrated in Fig. 10 depicts an example of the operations to analyze the biological information and provide the meal advice. Note that, the flowchart illustrated in Fig. 10 starts when the application installed on the mobile terminal 300 is launched.

First, the analysis request unit 29 determines whether the analysis request button provided by the application of the mobile terminal 300 has been operated (step S41). When the analysis request unit 29 has been operated, the analysis request unit 29 issues a request, to the server 400, for analyses on the biological information and the image of the meal recorded in the server 400 by the processing of the flowchart illustrated in Fig. 9 (step S42).

Upon receipt of the analysis request, the biological information analysis unit 34 of the server 400 analyzes the causal relationship between the meal content and the change between the pre-meal and post-meal biological information on the basis of the historical data stored in the storage unit 40 (step S44). Then, the advice provision unit 35 generates information of the meal advice on the basis of the analysis results from the biological information analysis unit 34, and sends that information to the mobile terminal 300 (step S45) .

The advice display unit 30 of the mobile terminal 300 displays the meal advice on the display of the mobile terminal 300 on the basis of the information of the meal advice received from the server 400 (step S46). Thus, the processing of the flowchart illustrated in Fig. 10 is ended.

As described in detail above, according to the third embodiment, not only the analysis of the change in the biological information (the carbohydrate indexes) measured by the biological information measurement apparatus 100, but also the meal advice corresponding to the analysis results obtained by analyzing the causal relationship with the meal content can be provided. This enables the user to be provided with meal advice that is more appropriate.

In order to provide the analysis and the meal advice described above, the historical data must be accumulated through measuring the biological information multiple times instead of only one time, and analyzing that historical data. According to the third embodiment, the message prompting the measurement of the biological information is notified when the user captures an image of a meal for some purpose, even if the user is not aware of the measuring of the biological information or the user forgets to measure the biological information. As a result, the user can perform the measurement of the biological information and reliably accumulate the historical data.

Note that, in the third embodiment, an example was described in which the analysis apparatus was configured from the mobile terminal 300 and the server 400, but the invention is not limited thereto. For example, the analysis apparatus may be configured from the mobile terminal 300 alone by providing the mobile terminal 300 with the functions of the server 400.

Additionally, in the third embodiment described above, an example is described in which the message prompting the measurement of the biological information is notified when the image of the meal is captured. However, this may be omitted. That is, the third embodiment was described as a configuration including additional functions over the second embodiment, but may have a configuration including additional functions over the first embodiment. In such cases, the functional blocks 25 to 27 of the mobile terminal 300 are omitted.

Note that, in the first to third embodiments described above, examples were described in which the biological information is measured once pre-meal and once post-meal, but the invention is not limited thereto. For example, the biological information may be measured once pre-meal and a plurality of times post-meal. In such cases, in the second and third embodiments, the measurement guidance unit 26 regularly notifies the message prompting the measurement of the biological information at timings such as one-hour after, two-hours after, and three-hours after the first message notification.

Additionally, in the first to third embodiments described above, examples were described in which the blood sugar level (the carbohydrate index) was measured as an example of the biological information, but the invention is not limited thereto. For example, the biological information measurement apparatus 100 may measure the blood hemoglobin level of the user as the biological information.

When measuring the carbohydrate index as in the embodiments described above, the analysis results and the meal advice thereof are information that is useful for dieting and health management. Managing meals to control carbohydrates is an important element in achieving a healthy diet. In contrast, the blood hemoglobin level is information that is useful for bodybuilding and the like.

For example, hemoglobin, which is the carrier of oxygen in red blood cells, has an important role in aerobic exercise such as marathon running and swimming. Higher levels of hemoglobin are advantageous when performing aerobic exercise. As such, by analyzing the change between pre-meal and post-meal hemoglobin levels, it is possible to analyze the correlation between the meal content and meal habits (eating speed, meal volume, and the like) and a change in the hemoglobin level and provide meal advice on eating methods and the like that help increase the hemoglobin level.

The first to third embodiments described above are all merely examples of embodiments for implementing the invention, and the technical scope of the invention should not be construed to be limited thereto. That is, the invention can be implemented in various forms without departing from the scope or main characteristics thereof.

### Reference Signs List

- 21: Biological information input unit
- 22: Biological information analysis unit
- 23: Advice provision unit
- 24: Captured image input unit
- 25: Image determination unit
- 26: Measurement guidance unit
- 27: Elapsed time measurement unit
- 28: Data sending unit
- 29: Analysis request unit
- 30: Advice display unit
- 31: Data receiving unit
- 32: Meal content analysis unit
- 33: History recording unit
- 34: Biological information analysis unit
- 35: Advice provision unit
- 40: Storage unit
- 100: Biological information measurement apparatus
- 200: Analysis apparatus
- 300: Mobile terminal (analysis apparatus)
- 400: Server (analysis apparatus)

## Claims

1. An analysis apparatus (200, 300, 400) that receives biological information measured by a biological information measurement apparatus (100) for measuring a blood sugar level or a blood hemoglobin level of a user as the biological information and analyses the biological information, the analysis apparatus (200, 300, 400) **characterized by** comprising:
a biological information input unit (21) that inputs the biological information measured before a meal and after the meal by the biological information measurement apparatus (100);
a biological information analysis unit (22, 34) that analyzes a change between pre-meal and post-meal biological information on the basis of pre-meal and post-meal biological information input by the biological information input unit (21) and times at which the pre-meal and post-meal biological information were obtained,
an advice provision unit (23) that provides meal-related advice on the basis of analysis results from the biological information analysis unit (22, 34),
a captured image input unit (24) that inputs a captured image captured by an imaging device when the user operates the imaging device;
an image determination unit (25) that analyzes the captured image input by the captured image input unit (24) and determines whether the captured image that has been input is an image of a meal; and
a measurement guidance unit (26), wherein when the image determination unit (25) determines that the captured image is an image of a meal, the measurement guidance unit (26) notifies a message prompting measurement of the biological information by the biological information measurement apparatus (100) when the determination is made and notifies the message again after a predetermined amount of time.

2. The analysis apparatus (200, 300, 400) according to claim 1, **characterized by** further comprising:
a meal content analysis unit (32) that analyzes meal content by image recognition processing or machine learning processing from the captured image input by the captured image input unit (24); and
a history recording unit (33) that associates and stores the pre-meal and post-meal biological information input by the biological information input unit (21), time information at which the pre-meal and post-meal biological information were obtained, and the meal content analyzed by the meal content analysis unit (32) as historical data in a storage unit (40); wherein
the biological information analysis unit (34) analyzes a tendency of a size of a gradient of the change between the pre-meal and post-meal biological information that will result from a particular meal content, or meal content with a strong tendency for producing a gradient steeper than a predetermined angle, or a degree of the amount of change between the pre-meal and post-meal biological information that will result from a particular meal content, or meal content with a strong tendency for increasing the amount of change of the biological information greater than a predetermined value, as a causal relationship between the meal content and the change between the pre-meal and post-meal biological information on the basis of the historical data stored in the storage unit (40) by the history recording unit (33) .

3. The analysis apparatus (200, 300, 400) according to claim 2, **characterized in that**
when the image determination unit (25) determines a captured image of the meal, the meal content analysis unit (32) analyzes the meal content from the captured image of the meal captured by the imaging device.

4. A meal advice provision system, **characterized by** comprising:
a biological information measurement apparatus(100) that measures a blood sugar level or a blood hemoglobin level as biological information of a user; and
an analysis apparatus(200, 300, 400) according to any one of claims 1 to 3.

## Patentansprüche

1. Analysevorrichtung (200, 300, 400), die von einer Biodaten-Messvorrichtung (100) zum Messen eines Blutzuckerspiegels oder eines Hämoglobinwerts gemessene Biodaten eines Benutzers als Biodaten empfängt und die Biodaten analysiert, **dadurch gekennzeichnet, dass** die Analysevorrichtung (200, 300, 400) Folgendes aufweist:
eine Biodaten-Eingabeeinheit (21), die Biodaten eingibt, die vor einer Mahlzeit und nach einer Mahlzeit von der Biodaten-Messvorrichtung (100) gemessen wurden;
eine Biodaten-Analyseeinheit (22, 34), die eine Veränderung zwischen Biodaten vor der Mahlzeit und nach der Mahlzeit auf Basis der Biodateneingabe vor und nach der Mahlzeit durch die Biodaten-Eingabeeinheit (21) analysiert und die Zeit erfasst, zu der die Biodaten vor der Mahlzeit und nach der Mahlzeit erhalten wurden;
eine Beratungseinheit (23), die speisenbezogene Empfehlungen auf Basis der Analyseergebnisse der Biodaten-Analyseeinheit (22, 34) bereitstellt;
eine Eingabeeinheit (24) für erfasste Bilder, die ein von einer Bildgebungsvorrichtung erfasstes Bild eingibt, wenn der Benutzer die Bildgebungsvorrichtung bedient;
eine Bildbestimmungseinheit (25), die das von der Eingabeeinheit (24) für erfasste Bilder erfasste Bild analysiert und feststellt, ob das eingegebene erfasste Bild ein Bild einer Mahlzeit ist; und
eine Messungsführungseinheit (26), wobei die Messungsführungseinheit (26) eine Nachricht, die zum Messen der Biodaten durch die Biodaten-Messvorrichtung (100) auffordert, ausgibt, wenn die Bildbestimmungseinheit (25) feststellt, dass das erfasste Bild ein Bild einer Mahlzeit ist, und die Nachricht nach einer vorbestimmten Zeitdauer erneut ausgibt.

2. Analysevorrichtung (200, 300, 400) nach Anspruch 1, **dadurch gekennzeichnet, dass** sie ferner Folgendes aufweist:
eine Speiseninhalt-Analyseeinheit (32), die Speiseninhalt durch auf Bilderkennung oder maschinelles Lernen ausgerichtete Verarbeitung aus dem erfassten Bild analysiert, das von der Eingabeeinheit (24) für erfasste Bilder eingegeben wurde; und
eine Aufzeichnungseinheit (33) für historische Werte, die Biodaten von vor der Mahlzeit und nach der Mahlzeit, die von der Biodaten-Eingabeeinheit (21) eingegeben wurden, Zeitangaben zum Erhalt der Biodaten von vor der Mahlzeit und nach der Mahlzeit und die Analyse des Speiseninhalts durch die Speiseninhalt-Analyseeinheit (32) als historische Daten in einer Speichereinheit (40) assoziiert und speichert;
wobei
die Biodaten-Analyseeinheit (34) eine Tendenz einer Größe eines Gradienten der Veränderung zwischen Biodaten von vor der Mahlzeit und nach der Mahlzeit, die sich aus einem bestimmten Speiseninhalt ergibt, oder Speiseninhalt mit einer starken Tendenz zu einem steileren Gradienten als ein vorbestimmter Winkel oder einen Grad der Betragsveränderung zwischen Biodaten von vor der Mahlzeit und nach der Mahlzeit, der sich aus einem bestimmten Speiseninhalt ergibt, oder Speiseninhalt mit einer starken Tendenz zum Erhöhen der Veränderung der Biodaten über einen vorbestimmten Wert hinaus als Kausalbeziehung zwischen Speiseninhalt und Veränderung zwischen Biodaten von vor der Mahlzeit und nach der Mahlzeit auf Basis der von der Aufzeichnungseinheit (33) für historische Werte in der Speichereinheit (40) gespeicherten historischen Daten analysiert.

3. Analysevorrichtung (200, 300, 400) nach Anspruch 2, **dadurch gekennzeichnet, dass** die Speiseninhalt-Analyseeinheit (32) den Speiseninhalt aus dem von der Bildgebungsvorrichtung erfassten Bild analysiert, wenn die Bildbestimmungseinheit (25) ein erfasstes Bild der Mahlzeit feststellt.

4. System zur Bereitstellung von Speisenempfehlungen, **dadurch gekennzeichnet, dass** es Folgendes aufweist:
eine Biodaten-Messvorrichtung (100), die einen Blutzuckerspiegel oder einen Hämoglobinwert als Biodaten eines Benutzers misst; und
eine Analysevorrichtung (200, 300, 400) nach einem der Ansprüche 1 bis 3.

## Revendications

1. Appareil d'analyse (200, 300, 400) qui reçoit des informations biologiques mesurées par un appareil de mesure d'informations biologiques (100) destiné à mesurer un taux de glycémie ou un taux d'hémoglobine dans le sang d'un utilisateur, en tant que des informations biologiques, et qui analyse les informations biologiques, l'appareil d'analyse (200, 300, 400) étant **caractérisé en ce qu'**il comprend :
une unité d'entrée d'informations biologiques (21) qui applique en entrée les informations biologiques mesurées, avant un repas, et après le repas, par l'appareil de mesure d'informations biologiques (100) ;
une unité d'analyse d'informations biologiques (22, 34) qui analyse un changement entre des informations biologiques d'avant repas et des informations biologiques d'après repas, sur la base des informations biologiques d'avant repas et des informations biologiques d'après repas appliquées en entrées par l'unité d'entrée d'informations biologiques (21), et des heures auxquelles les informations biologiques d'avant repas et d'après repas ont été obtenues ;
une unité de fourniture de conseils (23) qui fournit des conseils en matière de repas sur la base de résultats d'analyse provenant de l'unité d'analyse d'informations biologiques (22, 34) ;
une unité d'entrée d'image capturée (24) qui applique en entrée une image capturée qui est capturée par un dispositif d'imagerie lorsque l'utilisateur exploite le dispositif d'imagerie ;
une unité de détermination d'image (25) qui analyse l'image capturée appliquée en entrée par l'unité d'entrée d'image capturée (24) et détermine si limage capturée qui a été appliquée en entrée est une image d'un repas ; et
une unité d'orientation en matière de mesure (26), dans laquelle lorsque l'unité de détermination d'image (25) détermine que l'image capturée est une image d'un repas, l'unité d'orientation en matière de mesure (26) notifie un message sollicitant la mesure des informations biologiques par l'appareil de mesure d'informations biologiques (100) lorsque la détermination est effectuée, et notifie à nouveau le message après une période de temps prédéterminée.

2. Appareil d'analyse (200, 300, 400) selon la revendication 1, **caractérisé en ce qu'**il comprend en outre :
une unité d'analyse de contenu de repas (32) qui analyse un contenu de repas par le biais d'un traitement de reconnaissance d'image ou d'un traitement d'apprentissage machine, à partir de l'image capturée appliquée en entrée par l'unité d'entrée d'image capturée (24) ; et
une unité d'enregistrement d'historique (33) qui associe et stocke les informations biologiques d'avant repas et d'après repas appliquées en entrée par l'unité d'entrée d'informations biologiques (21), des informations sur l'heure à laquelle les informations biologiques d'avant repas et d'après repas ont été obtenues, et le contenu de repas analysé par l'unité d'analyse de contenu de repas (32), sous la forme de données historiques, dans une unité de stockage (40) ; dans lequel
l'unité d'analyse d'informations biologiques (34) analyse une tendance d'une taille d'un gradient du changement entre les informations biologiques d'avant repas et les informations biologiques d'après repas, qui résultera d'un contenu de repas particulier, ou d'un contenu de repas présentant une forte tendance à produire un gradient plus raide qu'un angle prédéterminé, ou un degré de la quantité de changement entre les informations biologiques d'avant repas et les informations biologiques d'après repas, qui résultera d'un contenu de repas particulier, ou d'un contenu de repas présentant une forte tendance à augmenter la quantité de changement des informations biologiques au-delà d'une valeur prédéterminée, en tant qu'une relation de cause à effet entre le contenu de repas et le changement entre les informations biologiques d'avant repas et les informations biologiques d'après repas, sur la base des données historiques stockées dans l'unité de stockage (40) par l'unité d'enregistrement d'historique (33).

3. Appareil d'analyse (200, 300, 400) selon la revendication 2, **caractérisé en ce que** :
lorsque l'unité de détermination d'image (25) détermine une image capturée du repas, l'unité d'analyse de contenu de repas (32) analyse le contenu de repas à partir de l'image capturée du repas, capturée par le dispositif d'imagerie.

4. Système de fourniture de conseils en matière de repas, **caractérisé en ce qu'**il comprend :
un appareil de mesure d'informations biologiques (100) qui mesure un taux de glycémie ou un taux d'hémoglobine dans le sang, en tant que des informations biologiques d'un utilisateur ; et
un appareil d'analyse (200, 300, 400) selon l'une quelconque des revendications 1 à 3.
